# FASCICULE DE BREVET EUROPEEN

(11) **EP 1 429 691 B1**
(45) Date de publication et mention de la délivrance du brevet: **11.06.2008**
(21) Numéro de dépôt: 02791881.2
(22) Date de dépôt: 26.09.2002
(51) Int. Cl.: A61F 2/36, A61F 2/30

(54) **PARTIE FEMORALE DE PROTHESE DE HANCHE**
FEMORALER HÜFTPROTHESETEIL
FEMORAL HIP PROSTHESIS PART

(30) Priorité: 28.09.2001 FR 0112552
(43) Date de publication de la demande: 23.06.2004
(73) Titulaire: Depuy (Ireland) Limited, Ringaskiddy, County Cork (IE); Badatcheff, Francois, 49000 Angers (FR); Besse, Jean-Pierre, 27400 Louviers (FR); Moretton, Jean-Claude, 88000 Chantraine (FR); Rochereau, Patrice, 32810 Duran (FR); Thumler, Peter, 40740 Dusseldorf (DE); Poitout, Dominique, 13009 Marseille (FR)
(72) Inventeur: BADATCHEFF, François, F-49000 Angers (FR); BESSE, Jean-Pierre, F-27400 Louviers (FR); MORETTON, Jean-Claude, F-88000 Chantraine (FR); ROCHEREAU, Patrice, F-32810 Duran (FR); THUMLER, Peter, 40740 Düsseldorf (DE); POITOUT, Dominique, F-13009 Marseille (FR); LAURENT-RENAUD, Nathalie, F-69006 Lyon (FR); PICON, Jean-Philippe, F-69007 Lyon (FR)
(74) Mandataire: Bernasconi, Jean Raymond
(86) Numéro de dépôt international: PCT/FR2002/003292
(87) Numéro de publication internationale: WO 2003/028594

(56) Documents cités:
- EP-A- 0 315 283
- EP-A- 0 808 618
- EP-A- 0 945 108
- EP-A- 1 025 817
- WO-A-92/00045
- WO-A-94/08534
- FR-A- 2 662 932
- FR-A- 2 729 292
- FR-A- 2 729 481
- FR-A- 2 735 970
- FR-A- 2 784 576
- US-A- 5 824 085

## Description

La présente invention a trait à un perfectionnement aux parties fémorales de hanches, du type qui comporte une queue, une partie métaphysaire, et une tête possédant, ou susceptible de recevoir une prothèse de tête fémorale, cette partie de prothèse fémorale étant destinée à être introduite et fixée dans le fémur après résection du col fémoral selon la procédure classique de pose de prothèses de col du fémur. Elle a également trait à un jeu de ces parties, destinées à fournir une pluralité de prothèses de tailles différentes pour s'adapter aux différences de taille des patients, ainsi qu'à un procédé de fabrication de la partie de prothèse fémorale selon l'invention et du jeu regroupant une pluralité de ces parties.

Le document EP-A-0 808 618 est l'état de la technique le plus proche et définit le préambule de la revendication 1.

De façon connue, une partie de prothèse fémorale comporte une queue de prothèse, destinée à être introduite dans le canal médullaire diaphysaire du fémur, suivie d'une partie métaphysaire de prothèse destinée à être fixée dans la métaphyse du fémur au-dessous du trait de résection du col, classiquement pratiqué avec un angle de 30° par rapport à l'axe du fémur, et une tête formant, ou destinée à recevoir, une rotule remplaçant la tête fémorale proprement dite qui a été réséquée, cette tête de prothèse étant le plus souvent en forme de prolongement tronconique destiné à recevoir une rotule de prothèse de tête, l'axe de cette tête étant convenablement incliné et présentant, par rapport au plan frontal anatomique, une antéversion de 8 à 20°, classiquement de 15°.

Afin d'essayer de s'adapter le mieux possible à la forme anatomique du réceptable métaphysaire formé par la corticale métaphysaire au-dessous du trait de résection, il est connu de donner, à la partie métaphysaire de la partie fémorale de prothèse, une hélitorsion permettant une meilleure adaptation à l'anatomie de la partie supérieure du fémur et destinée également à réduire, si possible, la perte de matière osseuse résultant de la mise en oeuvre de la râpe par le chirurgien avant la pose proprement dite. Cette hélitorsion, qui est classiquement comprise entre 5 et 10,° ou davantage, dans la direction de rotation interne, est obtenue en général soit par rotation progressive des plans successifs de section horizontale de partie métaphysaire de prothèse autour d'un axe géométrique, soit de façon empirique, par exemple par des procédés de moulage ou autres.

Ces prothèses connues ne donnent pas une entière satisfaction, notamment du fait qu'elles ne s'adaptent pas parfaitement à la surface interne de la corticale osseuse, ce qui peut créer des points de concentration de contraintes, avec toutes les conséquences qui en découlent, tant du point de vue de l'apparition de douleurs que de la tenue de la prothèse. Idéalement une tige fémorale, ou partie fémorale de prothèse de hanche, devrait ne venir en contact que de façon légère avec les différents points de la surface interne de la corticale métaphysaire tout en étant convenablement bloquée grâce à l'importance de la surface d'appui. Cette situation idéale est cependant particulièrement difficile à atteindre, en raison, notamment, du fait que, dans une population donnée dont l'anatomie fémorale est normale, et donc proche de la moyenne, il existe, néanmoins des différences dont l'effet s'amplifie dès lors que la forme extérieure de la surface métaphysaire de la partie de prothèse n'est pas convenablement conçue.

La présente invention se propose de remédier à ces inconvénients et de fournir une partie fémorale de prothèse de hanche qui minimise ou supprime les risques de mauvais contact et de création de contraintes exagérées au niveau de la métaphyse fémorale.

Un autre objectif de l'invention est de fournir une telle prothèse qui soit très bien adaptée à l'anatomie métaphysaire fémorale de la plupart des patients.

Un autre objectif de l'invention est de fournir une telle prothèse qui permette d'obtenir un tel appui en utilisant une râpe minimisant la perte de matière osseuse avant l'implantation.

Un autre objectif de l'invention est de fournir un jeu de prothèse selon l'invention permettant de s'adapter aux différences de tailles des patients, sachant que le passage d'une taille de prothèse à l'autre ne s'effectue pas de façon homothétique.

Un autre objectif de l'invention est de fournir un procédé de fabrication de ces prothèses et jeu de prothèses, qui puisse être mise en oeuvre de façon simple.

L'invention a pour objet une partie fémorale de prothèse comme définie dans la revendication 1.

Dans une forme de réalisation particulièrement préférée, l'hélitorsion n'est pas linéairement progressive, et s'étend, de préférence, d'une façon telle que si l'on considère sept plans de section géométrique équidistants consécutifs partant de la section métaphysaire inférieure à la section métaphysaire supérieure, qui forme la section métaphysaire proximale, les différences d'angles d'hélitorsion d'une section à l'autre, en partant du bas, sont, pour une hélitorsion de 8°, de 30', 30', 1°, 1°30', 2°, 2°30', chacune desdites valeurs pouvant varier de ± 30°. Les valeurs pour d'autres hélitorsions totales peuvent en être déduites de façon proportionnelle.

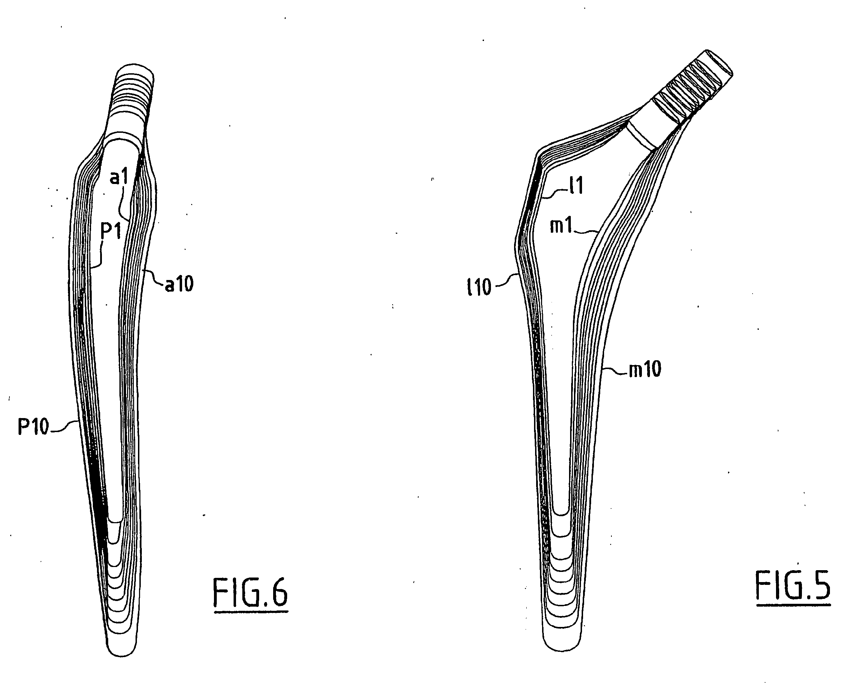

Dans une forme de réalisation particulièrement préférée, les quatre profils précités sont ceux qui apparaissent, selon les tailles de prothèse, sur les figures 5 et 6 annexées.

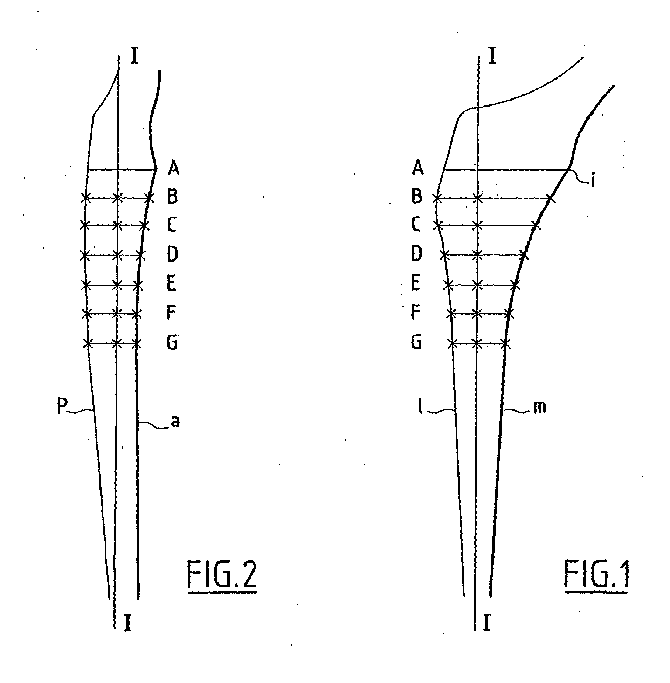

On obtient ainsi une forme tout à fait complexe de la partie métaphysaire, forme au demeurant parfaitement reproductible, et qui vient épouser, avec un maximum d'adaptation, la surface intérieure de la corticale fémorale d'un patient ayant une anatomie fémorale normale et ayant la taille correspondante. Bien entendu le nombre de différentes tailles peut être plus ou moins grand par rapport à la figure 1 et les profils correspondants peuvent alors en être convenablement extrapolés.

Dans une forme de réalisation préférée de l'invention, l'angle de convergence entre les deux grands côtés de chaque section géométrique est de l'ordre de 5 à 10°.

Il peut, avantageusement, varier avec la taille de la prothèse, par exemple en augmentant pour les prothèses plus grandes.

Les grands côtés de la section quadrilatérale arrondie peuvent présenter une partie centrale rectiligne, mais on peut également, en variante, prévoir des grands côtés courbes avec un grand angle de courbure.

Dans une forme de réalisation convenable de l'invention, ces grands rayons de courbure peuvent rester sensiblement constants au niveau de toutes les sections.

Dans une forme de réalisation avantageuse, la partie diaphysaire de la prothèse peut se prolonger, en continuité, avec la partie métaphysaire en se rétrécissant et en suivant une ligne centrale de courbure unique, déjà en soi connue dans l'anatomie fémorale, l'extrémité inférieure de cette tige intra-diaphysaire étant de préférence biseautée ou inclinée à sa partie postérieure, cette prothèse s'étendant ainsi sans double courbure, c'est-à-dire sans inversion de courbure.

La prothèse peut comporter sur sa partie métaphysaire et/ou sa partie diaphysaire des rainures facilitant l'accrochage et l'ostéo-intégration. Ces rainures peuvent avantageusement être horizontales dans la partie métaphysaire supérieure et longitudinales dans la partie inférieure de la partie métaphysaire et dans la queue de prothèse qui la suit. De façon avantageuse, la rainure horizontale supérieure peut déterminer, à son extrémité latérale, un point de repère que le chirurgien fera coïncider, lors de la pose, avec le bord le plus bas du plan de résection du col fémoral.

Dans une forme de réalisation particulièrement préférée, la ou les rainures longitudinales, qui peuvent se raccorder en courbe aux rainures horizontales inférieures, suit la forme anatomique de la queue de prothèse dans la partie métaphysaire inférieure et la partie diaphysaire, d'une façon telle que lors de l'introduction de la prothèse dans le fût fémoral, cette rainure produise un effet de guidage sans forcer sur la matière osseuse.

Selon un procédé de fabrication d'une prothèse on détermine, pour une taille donnée de la prothèse, quatre profils de projection de surfaces internes de corticale correspondantes, par exemple par radiographie ou par scanner, de préférence dans deux plans perpendiculaires, de préférence dans les plans sagittal et frontal qui donnent les profils latéral, médial, antérieur et postérieur. On détermine un quadrilatère, de préférence un rectangle, situé dans ce plan, ce rectangle étant, par exemple, aligné par son grand axe avec le plan final désiré d'hélitorsion. On détermine une succession équidistante de plans de section transversaux parallèles le long de la partie métaphysaire de prothèse dont le plan inférieur forme la section métaphysaire distale. On définit, dans chacun de ces plans, des quadrilatères dont les côtés restent respectivement parallèles, lesdits quadrilatères intersectant les différentes courbes de profil par leurs différents côtés dans le plan de niveau considéré. On inscrit, dans chacun des quadrilatères, une section de partie métaphysaire qui tangente les côtés du quadrilatère considéré, et dont les grands côtés sont inclinés, par rapport aux grands côtés de la section immédiatement supérieure, d'un angle d'hélitorsion donné, la sommation desdits angles d'hélitorsion depuis la section proximale jusqu'à la section distale déterminant l'angle total d'hélitorsion. On mémorise l'ensemble des données ainsi déterminées, et on pilote un dispositif de fabrication de têtes fémorales, ce dispositif pouvant être d'un type quelconque, par exemple par moulage, usinage ou forgeage.

Les calculs de détermination des données, leur stockage de mémorisation et leur utilisation pour piloter le dispositif de fabrication, peuvent mettre en oeuvre des moyens informatiques usuels, par exemple de CAO (conception assistée par ordinateur).

Pour fabriquer un jeu de ces prothèses, on met en oeuvre le procédé précité pour chaque prothèse individuelle, l'ensemble des prothèses du jeu étant déterminé par ce procédé en fonction de la taille qui détermine les profils de projection des surfaces corticales internes de la figure 1.

La prothèse peut être fabriquée en tout matériau convenable, par exemple en titane TA6V. Elle peut être revêtue en tout ou partie d'un revêtement favorisant l'ostéo-intégration, par exemple un revêtement d'hydroxy-apatite ou présenter ou non un état de surface particulier, comme cela est connu dans ce domaine.

Dans un mode de mise en oeuvre préféré du procédé de fabrication, la variation d'angle, par exemple pour un total d'une hélitorsion de 8°, est telle que si l'on considère sept plans équidistants allant du plan proximal au plan distal, la variation angulaire est plus importante vers le plan proximal que vers le plan distal, cette variation étant de préférence par incréments décroissants en allant du plan proximal au plan distal, un incrément étant compris, par exemple, entre 15' et 3°.

On préfère un angle d'hélitorsion de 8° par rapport au plan de référence déterminé par les condyles du fémur. Si l'on choisit un angle différent, de préférence compris entre 6 et 12°, par exemple 10°, les variations d'un plan à l'autre sont de préférence extrapolées.

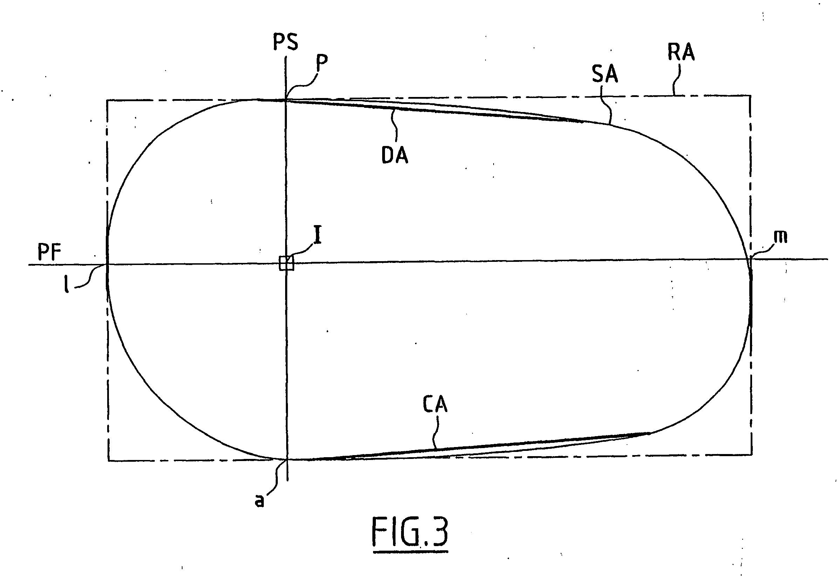

On peut avantageusement définir un axe géométrique formant un point dans le plan proximal, cet axe géométrique étant par exemple un axe diaphysaire sensiblement moyen. On peut, par exemple, repérer dans ce cas, les côtés du rectangle de section proximale par des valeurs Xₘₗ et Yₐₚ, comme représentés sur la figure 3, et formant les proportions du grand côté, respectivement du petit côté du rectangle, par rapport à la distance ml, c'est-à-dire la distance entre les points m et 1 dans le plan proximal, ce qui permet de faciliter la détermination du quadrilatère proximal pour chaque partie de prothèse, par exemple :
Xₘₗ = 45% ml
Yₐₚ = 28% ml

D'autres avantages et caractéristiques de l'invention apparaîtront à la lecture de la description suivante, faite à titre d'exemple non limitatif et se référant au dessin annexé dans lequel :
La figure 1 représente, à titre d'exemple, les deux profils latéral et médial de la surface interne de la corticale diaphysaire moyenne pour une taille particulière de prothèse dans le plan frontal.
La figure 2 représente les lignes directrices antérieure et postérieure dans le plan sagittal pour la même taille prothèse.
La figure 3 représente un exemple de section transversale dans le plan horizontal de la prothèse inscrite dans le quadrilatère défini au niveau de ce plan de section horizontale.
La figure 4 représente, en vue de dessus, cette section horizontale dans les sept plans de section correspondant aux figures 1 et 2.
La figure 5 représente les contours en projection dans le plan frontal, d'un jeu de dix prothèses de tailles différentes selon l'invention, dites tailles T1 à T7.
La figure 6 représente les contours de ce jeu en projection dans le plan sagittal.
Les figures 7 et 8 représentent des vues antérieure, respectivement postérieure d'une prothèse pour la taille intermédiaire, dite taille T4,5 pour une prothèse droite, dans le plan frontal.
La figure 9 représente une vue du côté médian dans la direction sagittale.
Les figures 10 à 16 représentent des sections transversales horizontales de la prothèse dans les plans A à G de la figure 7.

En se référant tout d'abord aux figures 1 et 2, on a représenté, pour une taille de prothèse donnée et donc pour une taille correspondante de fémur, les lignes de projection de la paroi interne du fût fémoral, ces projections formant les lignes médiane m et latérale 1 dans le plan frontal et les lignes antérieure a et postérieure p dans le plan sagittal. Ces lignes peuvent être déterminées par moyennage des projections sur les plans frontal, respectivement sagittal, d'un nombre significatif de cavités fémorales correspondant à une taille déterminée de fémur. Ceci peut être effectué, par exemple, à l'aide de radiographies sur une population statistiquement significative. La partie métaphysaire a été subdivisée par sept plans horizontaux A, B, C, D, E, F, G équidistants les uns des autres. L'intersection géométrique du plan supérieur A et de la ligne directrice médiale m, c'est-à-dire le point i, correspond sensiblement au point le plus bas du plan de résection du col fémoral par le chirurgien, lors de la pose d'une prothèse de hanche sur un patient dont la taille de fémur correspond sensiblement à celle représentée sur les figures. On a également représenté une droite géométrique I définie arbitrairement en une zone à peu près centrale du fût fémoral et qui intersecte les différentes sections A à G en un point appelé pôle de la section.

On doit comprendre, par ailleurs, que les lignes directrices m, l, a, p, pour d'autres tailles de fémurs, ne se déduisent pas homothétiquement des lignes des figures 1 et 2, mais doivent être définies sur une population statistiquement significative pour une autre taille de fémur.

Dix jeux de ces quatre lignes apparaissent sur les figures 5 et 6 pour dix tailles de fémurs, à savoir T1, T2, T3, T3,5, T4,5, T5, T5,5, T6 et T7.

En se référant à la figure 3, on a représenté un plan de coupe particulier, par exemple le plan de section A proximale sur lequel on voit les traces du plan frontal PF et du plan sagittal PS que l'on a fait passer par l'axe I arbitraire. On a représenté par m, 1, a et p, dans ce plan de section de la figure 3, les projections des lignes directrices sur les plans frontal et sagittal PF et PS. On définit, ainsi, un rectangle RA, dont les côtés sont parallèles aux plans PF et PS et qui passent par les points de projection m, 1, a, p. On pourrait, cependant, utiliser un quadrilatère différent, par exemple, un rectangle dont les côtés seraient un peu inclinés par rapport au plan PS et au plan PF, ou encore un trapèze ou un quadrilatère similaire.

Enfin, on a dessiné une courbe de section de prothèse SA, ayant une forme ovoïde à deux grands côtés, sensiblement aplatis et qui s'inscrit à l'intérieur du rectangle RA dont elle tangente les côtés, les points de tangences étant définis, de préférence, en considérant la section horizontale, dans ce plan, d'une population significative de fûts fémoraux, une telle allure de section étant assez proche des sections habituelles au niveau de ce plan des prothèses connues. On préfère que la section soit assez aplatie, mais non plate, sur les grands côtés et l'on a représenté par CA et DA les cordes géométriques qui sous-tendent les grands arcs des sections vues du côté antérieur et du côté postérieur.

Les cordes CA et DA font entre elles un petit angle, constant dans toutes les sections. Cet angle est, de préférence, dépendant de la taille de la prothèse, par exemple selon le tableau suivant :
Taille : T1, T2, T3, T3,5, T4, T4,5, T5, T5,5, T6, T7
Angle : 6°20', 7°20', 7°37', 7°53', 8°11', 8°14', 8°19', 8°24', 8°31', 8°40'.

Les deux petits côtés sont plus arrondis, les cordes qui sous-tendent les petits arcs, sur les petits côtés du rectangle géométrique, étant assez courtes.

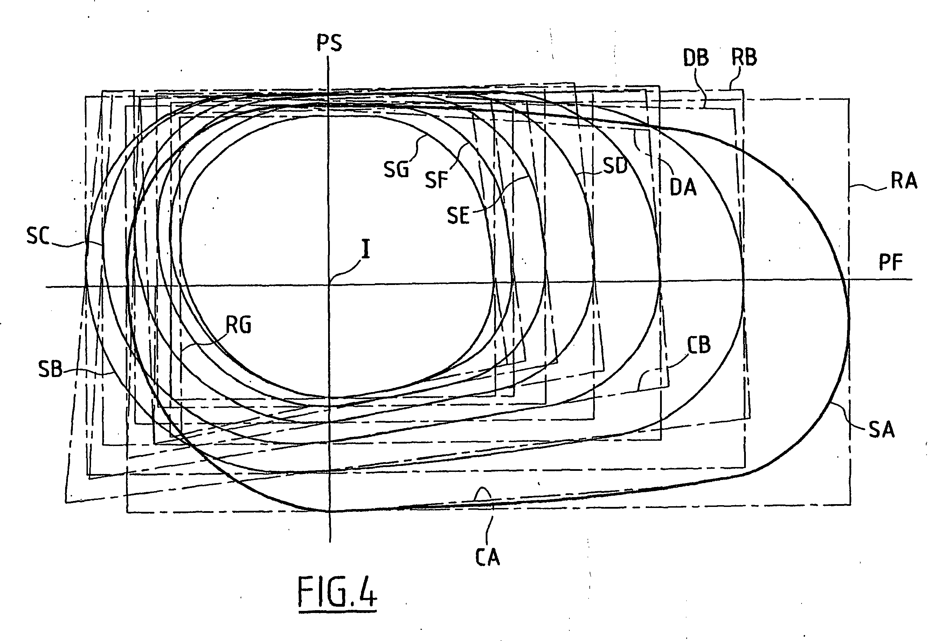

On se réfère maintenant à la figure 4.

Sur cette figure, on a représenté, en vue de dessus, les sept courbes ovoïdes de section SA à SG dans les différents plans de section A à G. La construction s'effectue de la façon suivante :

On trace, par exemple pour le plan de section B, un rectangle RB dont les côtés sont respectivement parallèles à ceux du rectangle RA, les côtés du rectangle RB passant respectivement par les intersections des lignes de profil m, 1, a, p dans le plan de section B. On trace, alors, dans le rectangle RB, une courbe de section de prothèse SB qui est inscrite de façon tangente dans le rectangle RB et dont l'orientation est telle que les cordes CB et DB qui sous-tendent ses deux grands côtés relativement aplatis, fassent avec les cordes CA et DA un angle d'hélitorsion correspondant à la variation d'hélitorsion désirée entre les plans A et B. Par exemple, l'angle préféré d'hélitorsion entre les cordes CA et CB, ainsi qu'entre les cordes DA et DB, est de 2°30', les cordes des deux petites faces étant de préférence décalées de la même variation d'hélitorsion quand on passe d'une section à l'autre. Bien entendu, on conserve à la courbe SB l'allure générale de la courbe SA avec cette double contrainte d'être inscrite dans le rectangle RB et d'avoir ses cordes des grandes faces décalées de l'angle susdit par rapport aux cordes du plan de section voisin A. On continue, ainsi, de proche en proche pour les sections consécutives, ce qui permet d'obtenir les différentes courbes de section SA à SG, telles que représentées sur la figure 4. Si l'on considère sept plans de section A à G déterminant six tranches de prothèses, les écarts d'hélitorsion d'un plan à l'autre, en partant du plan G et en direction du plan A, sont, pour une hélitorsion totale de 8° qui est la valeur préférée, de 30', 30', 1°, 1°30', 2°, 2°30'. De façon avantageuse, les valeurs et rayons de courbure des arcs aux quatre coins de la section varient en diminuant lorsque l'on s'éloigne de la section proximale A vers la section distale G, afin d'obtenir une progression harmonieuse des sections.

Il est ainsi possible d'obtenir une forme complexe de la partie métaphysaire de la prothèse qui peut être construite simplement à partir de l'adoption des lignes directrices m, 1, a, p et de la subdivision de la zone métaphysaire dans différentes sections, par exemple, 7 sections, ce nombre de sections pouvant être cependant légèrement supérieur ou légèrement inférieur, la construction de cette forme extrêmement complexe et particulièrement bien adaptée, étant cependant très simple. Elle peut même être effectuée par dessin manuel, en déterminant, manuellement, les différents rectangles RA à RG, en inscrivant, dans l'un des plans, une courbe de section de prothèse tangente et inscrite dans son rectangle, en optimisant cette courbe de façon empirique, comme cela est connu, en déterminant les cordes des grandes faces, et en déduisant les autres courbes de section conformément aux conditions précitées.

L'homme de l'art peut, évidemment, mettre au point, de façon simple, des algorithmes effectuant ces déterminations, ou conformes à ces déterminations effectuées manuellement, ces algorithmes étant alors utilisables pour piloter des machines-outils de fabrication de prothèses.

En se référant aux figures 5 et 6, on a représenté les projections dans les plans frontal, respectivement sagittal, d'un jeu de 10 prothèses de tailles différentes. On comprendra que les lignes d'encombrement extérieur d'une prothèse de taille donnée, par exemple la prothèse la plus grande, correspondent, du fait de la construction de la prothèse par le moyen défini précédemment, aux lignes de construction m, 1, a, p pour cette taille de prothèse. S'agissant d'une prothèse de taille dite 10, les lignes susdites sont appelées m₁₀, l₁₀, a₁₀, p₁₀. On a représenté, également, les courbes correspondantes pour la taille 1.

Il va de soi que les valeurs de longueur de queue de prothèse, de hauteur de partie métaphysaire, de longueur du col et éventuellement de dimension de la tête tronconique, varient comme cela est connu de l'homme du métier pour ce genre de prothèse.

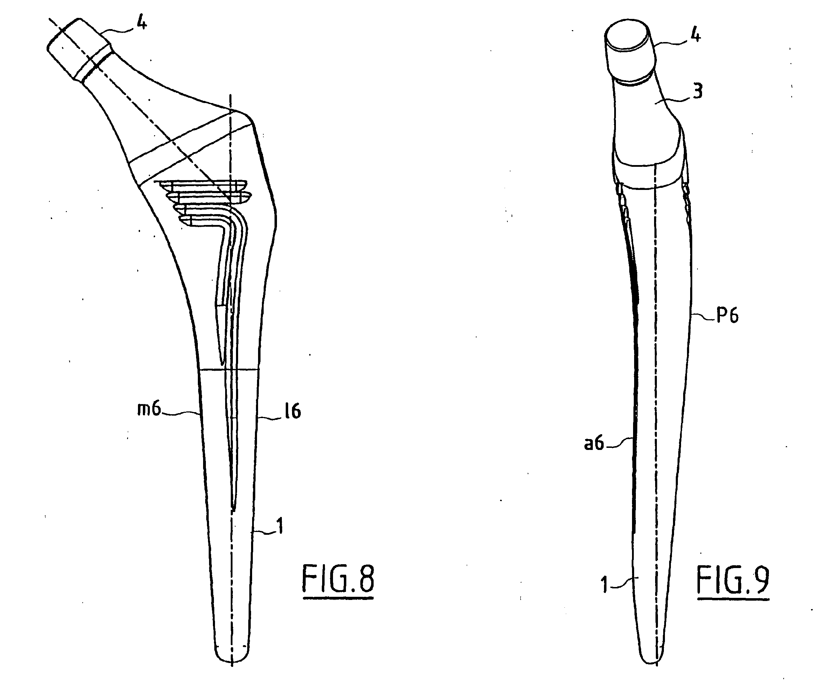

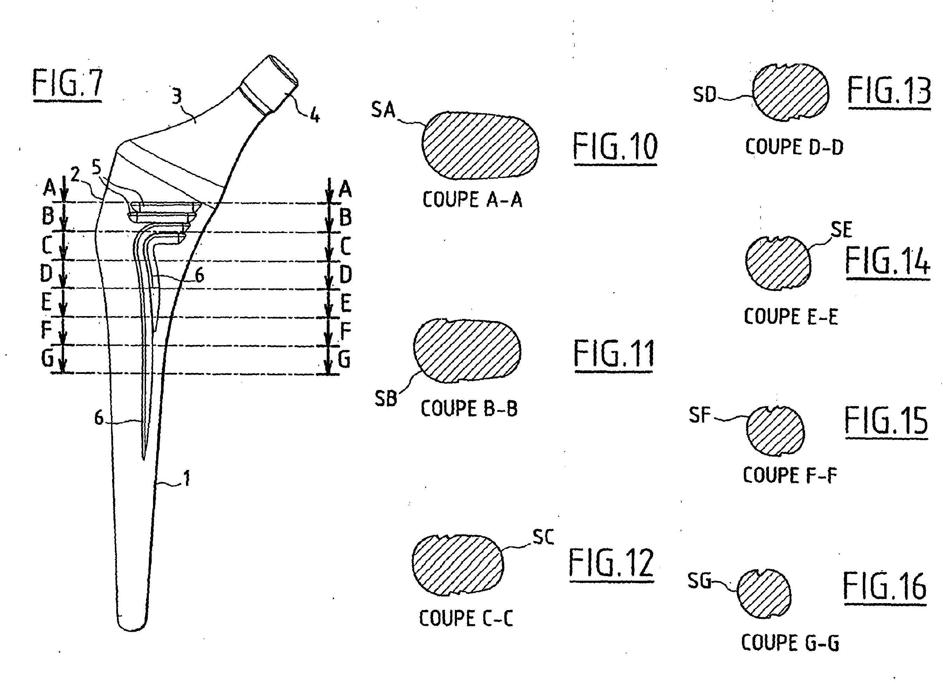

On se réfère, maintenant, aux figures 7 à 16.

La prothèse représentée est une prothèse de taille T4,5. Cette prothèse possède les dimensions suivantes :

La distance entre les plans de section A à G est de 51,6 mm.

La distance entre deux plans est donc de 8,6 mm.

La distance entre le plan A et le plan inférieur H à partir duquel débute l'arrondi terminal de la queue de prothèse est de 126,5 mm.

Les sections SA à SG sur les figures 10 à 16 obéissent aux règles qui ont été définies ci-dessus. La prothèse comporte de façon classique une queue de prothèse 1, une partie métaphysaire 2, un col 3, par exemple un col évasé et une tête tronconique 4 destinée à recevoir la rotule fémorale.

La prothèse peut avantageusement comporter un certain nombre de rainures. Ces rainures peuvent comporter plusieurs rainures horizontales 5, les rainures inférieures pouvant se poursuivre, après un décrochement courbe continu, par des rainures verticales 6. On voit les traces de ces rainures sur les figures 11 à 16. Les rainures verticales 6 sont avantageusement courbées et conformées de façon à suivre très exactement la direction de descente de la prothèse dans le fût fémoral au moment où la prothèse est suffisamment introduite pour prendre significativement contact avec la surface interne du fût préalablement traité avec une rape correspondante, ce qui forme un sorte de guidage terminal de la prothèse pendant la fin de son mouvement de descente et pendant l'impaction.

## Revendications

1. Partie fémorale de prothèse, du type comprenant une queue de prothèse, une partie métaphysaire de prothèse destinée à former une zone d'appui dans la métaphyse fémorale, et une tête ayant un angle convenable d'antéversion, ladite partie métaphysaire étant conformée pour présenter une hélitorsion, cette partie métaphysaire présentant une succession de sections géométriques (SA à SG) parallèles à un plan transversal par rapport à la direction générale de la prothèse, à des niveaux équidistants qui ont une forme de quadrilatère à sommets fortement arrondis avec deux grands côtés convergeant en direction médiale, chaque section étant inscrite, en y étant tangente sur quatre côtés, dans un quadrilatère géométrique situé dans le plan de ladite section au niveau considéré, lequel quadrilatère géométrique intercepte par ses quatre côtés, respectivement , quatre lignes géométriques (m, l, a, p) obtenues par projection, sur deux plans inclinés des profils de la surface interne de la corticale fémorale osseuse, lesdites sections pivotant l'une par rapport à l'autre en passant d'un plan équidistant au plan consécutif dans une direction angulaire en rotation interne depuis le bas vers le haut, l'ensemble de ces rotations aboutissant à l'angle d'hélitorsion souhaité, **caractérisée en ce que** l'angle total d'hélitorsion entre les plans extrêmes est de 8 à 12°, de préférence de 8 à 14°, et **en ce que** l'angle de convergence entre les deux grands côtés de chaque section géométrique est inférieure à 10°.

2. Partie fémorale de prothèse selon la revendication 1 **caractérisée en ce que** lesdits quadrilatères sont des rectangles (RA à RG).

3. Partie fémorale de prothèse selon l'une des revendications 1 et 2, **caractérisée en ce que** lesdites lignes (m, l, a, p) sont des profils de projection sur les plans frontal, respectivement sagittal, et forment ainsi les profils médial, latéral, antérieur et postérieur.

4. Partie fémorale de prothèse selon l'une des revendications 1 à 3, **caractérisée en ce que** les variations d'hélitorsion entre deux plans consécutifs est, dans le cas d'une division en sept plans, comprise entre 15' et 3°.

5. Partie fémorale de prothèse selon la revendication 4 **caractérisée en ce que** la variation d'hélitorsion est telle que, dans le cas d'une division en 7 plans consécutifs équidistants, et pour une hélirtorsion de 8°, les angles successifs d'hélitorsion sont de 30', 30', 1°, 1° 30', 2°, 2° 30', en direction proximale, chacune desdites valeurs pouvant varier de ± 30°.

6. Partie fémorale de prothèse selon l'une des revendications 1 à 5, **caractérisé en ce que** les grands côtés de la section quadrilatérale arrondie présentent des grands côtés courbes avec un grand angle de courbure.

7. Partie fémorale de prothèse selon la revendication 6 **caractérisée en ce que** ces grands rayons de courbure restent sensiblement constants au niveau de toutes les sections.

8. Partie fémorale de prothèse selon l'une des revendications 1 à 7, **caractérisée en ce qu'**elle comporte, sur sa partie métaphysaire et/ou sa partie diaphysaire, des rainures (5, 6) facilitant l'accrochage et l'ostéo-intégration, ces rainures étant horizontales dans la partie métaphysaire supérieure et longitudinales dans la partie inférieure de la partie métaphysaire et dans la queue de prothèse qui la suit.

9. Partie fémorale de prothèse selon la revendication 8, **caractérisée en ce que** la (ou les) rainure(s) longitudinale(s) suit la forme anatomique de la queue de prothèse dans la partie métaphysaire inférieure et la partie diaphysaire, d'une façon telle que lors de l'introduction de la prothèse dans le fût fémoral, cette rainure produise un effet de guidage sans forcer sur la matière osseuse.

## Claims

1. Femoral prosthesis part, of the type comprising a prosthesis shank, a metaphyseal prosthesis part for forming a bearing zone in the femoral metaphysis, and a head having a suitable anteversion angle, said metaphyseal part being shaped so as to display helitorsion and having a succession of geometrical portions (SA to SG) at equidistant levels parallel to a plane which is transverse relative to the general direction of the prosthesis, which portions are quadrilateral in shape with greatly rounded corners and two major medially-converging sides, each portion being inscribed in, and tangential on all four sides to, a geometrical quadrilateral located in the plane of said portion at the level in question, which geometrical quadrilateral intercepts, via its four sides, four geometrical lines respectively (m, l, a, p) which are obtained by projecting the profiles of the inner surface of the femoral bone cortex on two inclined planes, said portions rotating relative to one another in an inwardly-rotating angular direction on passing upwards from one equidistant plane to a consecutive plane, said rotations together resulting in the desired helitorsion angle, **characterised in that** the total helitorsion angle between the end planes is from 8 to 12°, preferably from 8 to 10°, and **in that** the angle of convergence between the two major sides of each geometrical portion is less than 10°.

2. Femoral prosthesis part according to claim 1, **characterised in that** said quadrilaterals are rectangles (RA to RG).

3. Femoral prosthesis part according to either claim 1 or claim 2, **characterised in that** said lines (m, l, a, p) arc profiles for projection onto the frontal and sagittal planes respectively, and therefore form medial, lateral, anterior, and posterior profiles.

4. Femoral prosthesis part according to any one of claims 1 to 3, **characterised in that** the variations in helitorsion between two consecutive planes is between 15' and 3° when subdivided into seven planes.

5. Femoral prosthesis part according to claim 4, **characterised in that** the variation in helitorsion is such that, when subdivided into seven consecutive equidistant planes, and for helitorsion of 8°, the successive helitorsion angles are 30', 30', 1°, 1° 30', 2°, 2° 30', in the proximal direction, each of said values being able to vary by ± 30°.

6. Femoral prosthesis part according to any one of claims 1 to 5, **characterised in that** the major sides of the rounded quadrilateral portion have major sides which are curved with a large angle of curvature.

7. Femoral prosthesis part according to claim 6, **characterised in that** said large radii of curvature remain substantially constant in all of the portions.

8. Femoral prosthesis part according to any one of claims 1 to 7, **characterised in that** it comprises grooves (5, 6) on its metaphyseal and/or diaphyseal part, the grooves (5, 6) acting to improve anchoring and osteo-integration and being horizontal in the upper, metaphyseal part and longitudinal in the lower metaphyseal part and in the following prosthesis shank.

9. Femoral prosthesis part according to claim 8, **characterised in that** the longitudinal groove(s) follow the anatomical shape of the prosthesis shank in the lower metaphyseal part and the diaphyseal part, in such a manner that during insertion of the prosthesis into the femoral shaft, said groove produces a guidance effect without applying force on the bone matter.

## Patentansprüche

1. Femoralprotheseteil mit einem Prothesenstiel und einem metaphysalen Protheseteil, der dazu bestimmt ist, eine Anlagezone in der Femoralmetaphyse zu bilden, und einem Kopf, der einen geeigneten Anteversionswinkel hat, wobei der metaphysale Teil dazu ausgebildet ist, eine Helitorsion zu bilden, wobei dieser metaphysale Teil eine Folge von geometrischen Schnitten (SA bis SG) in äquidistanten Höhen parallel zu einer Transversalebene in Bezug auf die Hauptrichtung der Prothese aufweist, die eine viereckige Form mit stark abgerundeten Ecken und mit zwei in Medialrichtung konvergierenden großen Seiten haben, wobei jeder Schnitt, indem er es an vier Seiten tangiert, in ein geometrisches Viereck einbeschrieben ist, das in der Ebene des genannten Schnittes in der betrachteten Höhe liegt, welches geometrische Viereck mit seinen vier Seiten vier jeweilige geometrische Linien (m, l, a, p) abfängt, die durch Projektion von Profilen der inneren Oberfläche der Oberschenkelknochenrinde auf zwei geneigte Ebenen erhalten werden, wobei die genannten Schnitte gegeneinander verschwenkt sind, indem sie von einer äquidistanten Ebene in einer Winkelrichtung einer Einwärtsdrehung von unten nach oben in die nachfolgende Ebene übergehen, wobei die Gesamtheit dieser Drehungen zu dem gewünschten Helitorsionswinkel führt, **dadurch gekennzeichnet, daß** der Gesamtwinkel der Helitorsion zwischen den endständigen Ebenen 8 bis 12°, vorzugsweise bis 8 bis 10° beträgt, und daß der Konvergenzwinkel zwischen den beiden großen Seiten jedes geometrischen Schnittes kleiner als 10° ist.

2. Femoralprotheseteil nach Anspruch 1, **dadurch gekennzeichnet, daß** die genannten Vierecke Rechtecke (RA-RB) sind.

3. Femoralprotheseteil nach Anspruch 1 oder 2, **dadurch gekennzeichnet, daß** die genannten Linien (m, l, a, p) Projektionsprofile auf die Frontal- bzw. Sagittalebene sind und somit die Medial-, Lateral-, Anterior- und Posteriorprofile bilden.

4. Femoralprotheseteil nach einem der Ansprüche 1 bis 3, **dadurch gekennzeichnet, daß** die Variationen der Helitorsion zwischen zwei aufeinanderfolgenden Ebenen, im Fall einer Aufteilung in sieben Ebenen, zwischen 15' und 3° beträgt.

5. Femoralprotheseteil nach Anspruch 4, **dadurch gekennzeichnet, daß** die Variation der Helitorsion so ist, das im Fall einer Aufteilung in 7 aufeinanderfolgende äquidistante Ebenen und für eine Helitorsion von 8°, die aufeinanderfolgenden Helitorsionswinkel in Proximalrichtung 30', 30', 1°, 1°30', 2°, 2°30' betragen, wobei die genannten Werte jeweils um ± 30° variieren können.

6. Femoralprotheseteil nach einem der Ansprüche 1 bis 5, **dadurch gekennzeichnet, daß** die großen Seiten des abgerundeten viereckigen Schnittes gekrümmte große Seiten mit einem großen Krümmungswinkel bilden.

7. Femoralprotheseteil nach einem Anspruch 6, **dadurch gekennzeichnet, daß** diese großen Krümmungsradien bei all den Schnitten im wesentlichen konstant bleiben.

8. Femoralprotheseteil nach einem der Ansprüche 1 bis 7, **dadurch gekennzeichnet, daß** es an dem metaphysalen Teil und/oder seinem diaphysalen Teil Nuten (5, 6) aufweist, die die Verankerung und Osteointegration erleichtern, wobei diese Nuten in dem oberen metaphysalen Teil horizontal und in dem unteren metaphysalen Teil und dem sich daran anschließenden Prothesestiel longitudinal sind.

9. Femoralprotheseteil nach Anspruch 8, **dadurch gekennzeichnet, daß** dielontitudinale(n) Nut oder Nuten der anatomischen Form des Prothesestiels in dem unteren metaphysalen Teil und dem diaphysalen Teil folgen, derart, daß beim Einführen der Prothese in den Femoralschaft diese Nut einen Führungseffekt erzeugt ohne auf das Knochenmaterial zu drücken.
